# FASCICULE DE BREVET EUROPEEN

(11) **EP 2 844 691 B1**
(45) Date de publication et mention de la délivrance du brevet: **14.08.2019**
(21) Numéro de dépôt: 13727244.9
(22) Date de dépôt: 02.05.2013
(51) Int. Cl.: C08K 5/00, C08L 27/06, C08K 5/10, C08K 5/1535, C07D 493/04, C07C 69/80, C07C 69/003, C07C 69/74, C07C 69/75

(54) **COMPOSITIONS PLASTIFIANTES COMPRENANT DES ACCELERATEURS DE GELIFICATION A BASE D'ESTER DE 1,4 : 3,6-DIANHYDROHEXITOL DE FAIBLE POIDS MOLAIRE**
WEICHMACHERZUSAMMENSETZUNGEN MIT GELIERUNGSBESCHLEUNIGERN AUF BASIS EINES ODER MEHRERER ESTER VON 1,4: 3,6-DIANHYDROHEXITOL MIT NIEDRIGEM MOLEKULARGEWICHT
PLASTICISER COMPOSITIONS COMPRISING GELIFICATION ACCELERATORS BASED ON ESTER(S) OF 1,4 : 3,6-DIANHYDROHEXITOL HAVING LOW MOLAR WEIGHT

(30) Priorité: 03.05.2012 FR 1254086
(43) Date de publication de la demande: 11.03.2015
(73) Titulaire: Roquette Frères, 62136 Lestrem (FR)
(72) Inventeur: FERON, Thierry, F-62232 Fouquieres Les Bethune (FR); SOBOCINSKI, Monique, F-62136 La Couture (FR); WYART, Hervé, F-62149 Cuinchy (FR); BREITSCHEIDEL, Boris, 67165 Waldsee (DE); WAGNER, Jochen, 66957 Ruppertsweiler (DE)
(74) Mandataire: Cabinet Plasseraud
(86) Numéro de dépôt international: PCT/FR2013/050967
(87) Numéro de publication internationale: WO 2013/164545

(56) Documents cités:
- WO-A1-99/45060
- DE-A1-102010 002 856
- US-A1- 2007 027 242
- US-A1- 2009 301 348

## Description

### Domaine de l'invention

L'invention a pour objet une composition, utile comme plastifiant de polymères, comprenant au moins un ester de 1,4 :3,6 dianhydrohexitol et un second composé ayant fonction de plastifiant. Un autre aspect de l'invention porte sur l'utilisation de cet ester de 1,4 :3,6 dianhydrohexitol comme accélérateur de gélification de polymères.

### Etat de la technique

Du fait de leurs nombreux avantages, l'utilisation de polymères synthétiques s'est généralisée dans de nombreuses applications depuis le siècle dernier.

Cependant, ces polymères peuvent présenter des inconvénients, comme en particulier, leurs propriétés mécaniques qui peuvent être insuffisantes pour certaines utilisations. Par exemple, ils peuvent présenter un allongement à la rupture très faible à température ambiante ou être peu résistants au choc.

De plus, il peut être nécessaire de modifier le comportement en phase fondue de ces polymères, notamment pour pouvoir les mettre en oeuvre dans des procédés de transformation de type enduction ou encore en calandrage. En d'autres termes, il est nécessaire que le polymère ait dépassé sa température de fusion, ou encore sa température de gélification, et qu'ainsi le polymère présente, dans cet état gélifié, une viscosité adaptée au procédé de mise en forme pour pouvoir être correctement transformé.

Pour pouvoir les utiliser dans des applications plus variées, il est également nécessaire de modifier les propriétés de ces polymères, par exemple pour les rendre plus souples, plus résistants au choc ou encore leur permettre d'avoir un aspect plus doux.

Pour ceci, ces polymères peuvent être mélangés avec des « plastifiants ».

Par « plastifiant », on entend tout produit qui, lorsqu'il est mélangé en quantité suffisante avec un polymère, a pour fonction de diminuer la température de transition vitreuse dudit polymère.

En diminuant la température de transition vitreuse du polymère, la souplesse de ce dernier est augmentée et les propriétés mécaniques de ce polymère plastifié sont modifiées. Ainsi, en ajoutant un plastifiant à une composition de polymère, on observe généralement une diminution du module d'Young, une diminution de la contrainte à la rupture et/ou une augmentation de la déformation à la rupture.

Ces propriétés modifiées du polymère lui permettent alors d'être utilisé dans des applications plus variées, par exemple dans des films ou des feuilles souples.

Lors du procédé de mise en oeuvre de la matière plastique, les plastifiants sont généralement mélangés au polymère, ce qui permet la diminution de la température de ramollissement du polymère.

Ce mélange peut se faire par différents procédés de mise en oeuvre.

Dans le cas des polychlorures de vinyle (PVC) par exemple, le polymère peut être transformé en objet par différentes techniques de transformation des matériaux thermoplastiques, et en particulier par extrusion, par calandrage ou encore par enduction via un procédé de type plastisol.

Afin d'obtenir ce mélange thermoplastique, on mélange le PVC avec le plastifiant en apportant à ce système de l'énergie, sous forme de température et d'énergie mécanique. Dans le cas de l'extrusion ou du malaxage, ce mélange se fait dans un système fermé. Dans le cas d'un mélange sur cylindres, ce mélange se fait dans un système ouvert. Le polymère peut être ensuite mis en forme, par exemple par des procédés de thermoformage ou de calandrage. Généralement, on réalise une étape de mélange à sec (en anglais *dry blend*) avant l'étape de mélange thermomécanique.

Selon le procédé plastisol, on réalise généralement un mélange pour former une pâte de PVC, cette pâte est ensuite mise en forme par une étape d'enduction ou de moulage dans laquelle la pâte est chauffée dans un four pour former la pièce.

Quel que soit le procédé, il faut que le polymère soit correctement fondu ou gélifié pour pouvoir être mis en forme de manière satisfaisante et ainsi faire en sorte que l'objet formé à la fin du procédé ait de bonnes propriétés.

Pour tous ces procédés d'obtention de mélanges thermoplastiques à partir de PVC, on utilise généralement des plastifiants de la famille des esters phtaliques. Il s'agit encore à ce jour très généralement de phtalate de dioctyle ou de phtalate de diisononyle. Ces plastifiants sont très efficaces pour la plastification de polymères, et sont facilement disponibles sur le marché, pour un coût relativement faible.

Cependant, du fait des problèmes de toxicité des phtalates, d'autres plastifiants ont également été développés ces dernières années, comme l'acide polycarboxylique cyclohexane et ses dérivés, qui ont fait l'objet des demandes de brevet WO 00/78853 et WO 99/32427. A titre d'exemple, on peut citer l'ester de diisononyle d'acide dicarboxylique 1,2-cyclohexane commercialisé par BASF sous la marque Hexamoll®.

Comme autre plastifiant, on peut également citer les dérivés esters de glycérol, tel que le Grindsted® obtenu à partir de glycérol et d'huile de ricin et commercialisé par la société Danisco. Ces plastifiants présentent l'avantage d'être obtenus à partir de produits biosourcés.

L'utilisation de dérivés de 1,4 :3,6 dianhydrohexitols comme plastifiants de polymères a déjà été décrite dans le document WO 99/45060. Ces dérivés ne présentent pas les problèmes de toxicité des phtalates. Ces plastifiants présentent en outre l'avantage d'être au moins partiellement biosourcés.

Les propriétés mécaniques des polymères plastifiés avec ces dérivés sont excellentes, proches de celles obtenues avec les plastifiants de type phtalates.

Dans le cadre de ses recherches, la Société Demanderesse a constaté que certains de ces composés présentent l'inconvénient de plastifier les polymères relativement lentement, ce qui nécessite l'utilisation d'une énergie thermomécanique plus importante pour la mise en forme du polymère, Cela implique également des temps de transformation importants et ainsi une perte de productivité.

Ce même problème est également observé lorsque l'on utilise comme plastifiants les esters phtaliques, les dérivés d'acide polycyclohexane et les dérivés esters de glycérol déjà cités. Pour répondre à ce problème de vitesse lente de plastification, on utilise généralement dans le procédé de mise en oeuvre des accélérateurs de gélification, encore appelés accélérateurs de plastification et bien connus de l'homme du métier sous le terme « fast fuser ». Ces composés sont généralement utilisés en combinaison avec les plastifiants dans le procédé de mise en oeuvre.

Sans être liée à une quelconque théorie, la Demanderesse explique l'effet d'accélération de la gélification, par le fait que la substance pénètre rapidement dans le polymère et s'intercale entre les chaines de ce polymère. Le réseau des molécules de polymère est donc rendu très rapidement plus « lâche », ce qui permet une gélification rapide et une introduction du plastifiant plus facile entre les chaînes du polymère et ce qui a pour effet d'obtenir un polymère plastifié plus rapidement.

La conséquence pour le procédé est que le polymère peut être ainsi transformé plus vite, et/ou avec moins d'énergie thermomécanique.

Comme exemple d'accélérateur de gélification déjà connu, on peut citer le phtalate de diisobutyle (DIBP), la triacétine ou encore les produits commerciaux JAYFLEX™ MB 10 (monobenzoate d'isodécyle) commercialisé par la société Exxon Mobil ou SANTICIZER® 9500 (monobenzoate de 2-ethyl hexyle) commercialisé par la société Ferro.

Un inconvénient de ces accélérateurs est que leur utilisation induit généralement un dégagement de composés organiques volatils (ci-après COV) relativement important lors de la mise en oeuvre.

Ce dégagement est d'autant plus important dans les procédés utilisant un système de mélange ouvert, tels que les procédés de calandrage ou les procédés d'enduction de plastisol, car des quantités importantes de matières volatiles sont émises lors de la fabrication de produits selon ces modes de mise en oeuvre.

Par ailleurs, on a pu observer que les accélérateurs de gélification généralement utilisés avaient, lors de l'utilisation de l'objet formé à partir de la matière plastique, tendance à « migrer » hors de l'objet formé. On dit également que le produit exsude. Il en résulte un vieillissement UV et thermique accéléré du plastique formé, ce qui a pour conséquence d'en diminuer les propriétés mécaniques. La surface de cet objet émet ainsi des polluants, ce qui est encore plus problématique lorsqu'il s'agit d'un emballage et que le contenu est pollué par ces substances ou encore lorsque les objets sont placés en intérieur, particulièrement dans les crèches ou les hôpitaux.

Le document US 2007/0027242 décrit à l'exemple un mélange de phtalates, utile comme plastifiant, ce mélange comprenant des esters d'acide phtalique et d'alcools saturés à 7 atomes de carbone (de masse molaire égale à 362 g.mol⁻¹) ainsi que des esters d'acide phtalique et d'alcools saturés à 10 atomes de carbone (de masse molaire égale à 446 g.mol⁻¹). L'ester fabriqué à partir des alcools saturés à 7 atomes de carbone est utilisé comme un accélérateur de gélification. L'avantage avancé pour cette composition est qu'elle est peu volatile, par rapport à des compositions comprenant comme accélérateur de gélification les plus courants, par exemple des phtalates de dibutyle ou d'isobutyle. Toutefois, ce mélange plastifiant n'est pas totalement satisfaisant en termes de vitesse de plastification. De plus, la Demanderesse a même pu vérifier (voir exemples) que l'ester phtalique en C7 est très peu efficace en tant qu'accélérateur de gélification lorsqu'il est associé à d'autres types de plastifiants que les phtalates, par exemple à un diester de 1,4 : 3,6-dianhydrohexitol ou à un ester de l'acide polycarboxylique cyclohexane.

Il reste donc nécessaire de trouver de nouveaux procédés, utilisant des composés particuliers, qui permettent de résoudre au mieux l'ensemble de ces problèmes et qui constituent un excellent compromis des propriétés déjà décrites.

Enfin, certains de ces plastifiants, en particulier certains dérivés de 1,4 : 3,6-dianhydrohexitol peuvent présenter des températures de congélation proches de 0°C. Lors du stockage de ces plastifiants, qui peut se faire à l'extérieur et donc à des températures négatives très basses, cette température de congélation peut être problématique, voire rédhibitoire car le plastifiant est alors difficilement manipulable.

Afin de permettre le stockage de ces plastifiants même dans des conditions météorologiques défavorables, il est donc nécessaire de trouver des compositions, aptes à plastifier des polymères, qui restent liquides, même à des températures atteignant -10°C, voire -15°C.

Ainsi, outre de nouveaux procédés, il est également nécessaire de trouver de nouvelles compositions qui permettent de résoudre au mieux l'ensemble de des problèmes susmentionnés.

### Résumé de l'invention

La Demanderesse a eu le mérite de trouver cette composition particulière permettant de plastifier de manière très efficace les polymères, tout en permettant de répondre aux différents problèmes précédemment décrits.

Cette composition plastifiante comprend un composé (A) agissant comme un accélérateur de gélification et un composé (B) agissant comme un plastifiant.

L'invention a ainsi pour objet une composition comprenant :
- de 0,1 à 99% en masse d'au moins un ester de 1,4 : 3,6-dianhydrohexitol (A) dont la masse molaire va de 255 à 345 g.mol⁻¹, et choisi parmi les monoesters et les diesters d'isosorbide, d'isomannide et d'isoidide ;
- de 1 à 99,9% en masse d'au moins un composé (B) dont la masse molaire est supérieure à 345 g.mol⁻¹ et choisi parmi :
   ▪ les esters de 1,4 :3,6-dianhydrohexitol choisis parmi les monoesters et les diesters d'isosorbide, d'isomannide et d'isoidide et dont les groupes esters de 1,4 :3,6-dianhydrohexitol sont des groupes comprenant de 1 à 24 atomes de carbone, de préférence des groupes comprenant de 6 à 12 atomes de carbone ;
   ▪ les esters de l'acide polycarboxylique cyclohexane ;
   ▪ les esters de l'acide phtalique.

La composition selon l'invention, selon certaines variantes, a en outre pour avantage de présenter, une meilleure tenue au froid que les compositions de plastifiant déjà connues. Ceci est particulièrement vrai pour les compositions où le composé (B) est un ester de 1,4 :3,6-dianhydrohexitol.

L'invention porte également sur un procédé de fabrication d'un objet à base d'une composition de polymère plastifié comprenant un polymère (C) et la composition contenant (A) et (B) selon l'invention. Ce procédé comprend les étapes suivantes :
- une étape de sélection de l'ester (A), du composé (B) et du polymère (C) ;
- une étape d'introduction des constituants (A), (B) et (C) dans un système mélangeur, (A) et (B) étant introduits dans les proportions telles que définies dans la composition de l'invention ;
- une étape de mélange des constituants (A), (B) et (C) ;
- une étape de chauffage de ce mélange ;
- une étape de mise en forme du mélange sous la forme d'unobjet ;
- enfin une étape de récupération dudit objet comprenant la composition de polymère plastifié ;
l'étape d'introduction des constituants (A), (B) et (C) dans le mélangeur pouvant se faire séparément ou par l'intermédiaire d'un mélange de constituants, et de manière simultanée ou séquencée ; les étapes de mélange et de chauffage pouvant être réalisées de manière simultanée ou de manière séquencée.

L'invention va maintenant être détaillée dans la suite de la description.

### Brève description des figures

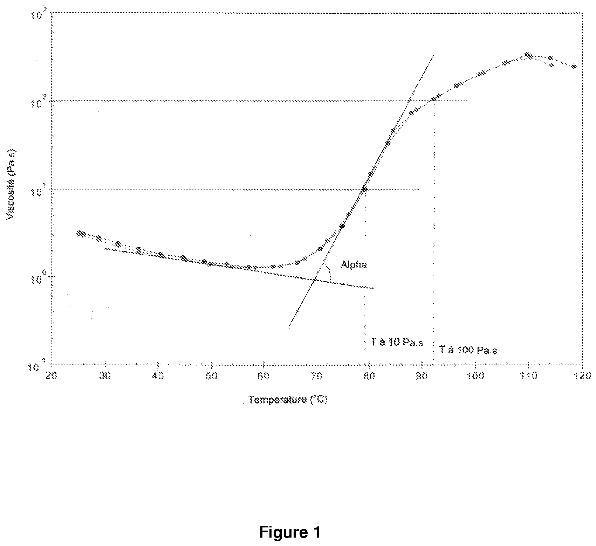

La Figure 1 indique l'évolution de la viscosité en fonction de la température d'une pâte de polymère comprenant un polychlorure de vinyle et un accélérateur de gélification.

### Description détaillée de l'invention

Le procédé et la composition selon l'invention utilisent tous deux les composés (A) et (B) qui vont maintenant être décrits en détail.

Le composé (A) est un ester de 1,4 : 3,6-dianhydrohexitol (A) dont la masse molaire va de 255 à 345 g.mol⁻¹, et choisi parmi les monoesters et les diesters d'isosorbide, d'isomannide ou d'isoidide.

Les groupes esters de l'ester (A) sont choisis pour que la masse molaire de (A) aille de 255 à 345 g.mol⁻¹. Ce groupe ester peut être issu d'un acide carboxylique, c'est-à-dire susceptible d'être obtenu par réaction d'un acide carboxylique avec une fonction alcool du 1,4 : 3,6-dianhydrohexitol. Par exemple, si l'acide est de l'acide valérique, le groupe ester est un groupe valérate. Avantageusement, les groupes esters de 1,4 : 3,6-dianhydrohexitol sont des groupes comprenant de 2 à 8 atomes de carbone, c'est-à-dire que l'acide réagissant avec le 1,4 : 3,6-dianhydrohexitol comprend de 2 à 8 atomes de carbone.

Préférentiellement, le groupe ester de l'ester (A) est un groupe alkyle, c'est-à-dire que l'ester est obtenu par réaction de 1,4 :3,6-dianhydrohexitol avec un ou plusieurs monoacides carboxyliques saturés. Le groupe alkyle peut être un groupe cycloalkyle, alkyle linéaire ou alkyle ramifié. Préférentiellement, le groupe alkyle est linéaire ou ramifié, tout préférentiellement linéaire.

Cet ester (A) peut être choisi parmi les dipropionate de 1,4 : 3,6-dianhydrohexitol, les dibutyrate de 1,4 : 3,6-dianhydrohexitol, les diisobutyrate de 1,4 : 3,6-dianhydrohexitol, les divalérate de 1,4 : 3,6-dianhydrohexitol, les diisovalérate de 1,4 : 3,6-dianhydrohexitol, les dihexanoate de 1,4 : 3,6-dianhydrohexitol, les propionate butyrate de 1,4 : 3,6-dianhydrohexitol, le propionate isobutyrate de 1,4 : 3,6-dianhydrohexitol, les propionate valérate de 1,4 : 3,6-dianhydrohexitol, les propionate isovalérate de 1,4 : 3,6-dianhydrohexitol, les propionate hexanoate de 1,4 : 3,6-dianhydrohexitol, les butyrate isobutyrate de 1,4 : 3,6-dianhydrohexitol, les butyrate valérate de 1,4 : 3,6-dianhydrohexitol, les butyrate isovalérate de 1,4 : 3,6-dianhydrohexitol, le butyrate hexanoate de 1,4 : 3,6-dianhydrohexitol, les isobutyrate valérate de 1,4 : 3,6-dianhydrohexitol, les isobutyrate isovalérate de 1,4 : 3,6-dianhydrohexitol, les isobutyrate hexanoate de 1,4 : 3,6-dianhydrohexitol, les valérate hexanoate de 1,4 : 3,6-dianhydrohexitol et les isovalérate hexanoate de 1,4 : 3,6-dianhydrohexitol.

Tout préférentiellement, l'ester (A) est un divalérate de 1,4 : 3,6-dianhydrohexitol ou un dihexanoate de 1,4 : 3,6-dianhydrohexitol, préférentiellement un divalérate de 1,4 : 3,6-dianhydrohexitol.

Préférentiellement, l'ester de 1,4 : 3,6-dianhydrohexitol (A) est un ester d'isosorbide.

Ces esters dont les groupes esters sont des groupes alkyles sont particulièrement efficaces, en particulier dans leurs variantes préférées. Comme l'isomannide, l'isoidide et l'isosorbide peuvent être obtenus respectivement à partir du mannitol, de l'iditol et du sorbitol, qui sont eux-mêmes obtenus à partir d'amidon, les esters de 1,4 : 3,6-dianhydrohexitol utiles à l'invention présentent en outre l'avantage d'être partiellement biosourcés, voire totalement biosourcés si on utilise un acide également biosourcé.

La composition selon l'invention comprend au moins un ester (A), c'est-à-dire qu'elle peut comprendre un mélange d'esters (A) décrits ci-dessus.

Ces esters sont connus et peuvent être obtenus par une réaction d'estérification de 1,4 : 3,6-dianhydrohexitol avec au moins un acide carboxylique. Cet acide carboxylique peut être l'acide éthanoïque, l'acide propionique, l'acide butyrique, l'acide isobutyrique, l'acide valérique, l'acide isovalérique, l'acide hexanoïque, l'acide heptanoïque et l'acide octanoïque. La synthèse de ces esters est présentée par exemple dans le document WO 99/45060 déjà cité.

Selon l'invention, le composé (B) est un composé dont la masse molaire est supérieure à 345 g.mol⁻¹ et qui est choisi parmi :
- les esters de 1,4 :3,6-dianhydrohexitol, choisi parmi les monoesters et les diesters d'isosorbide, d'isomannide et d'isoidide et dont les groupes esters de 1,4 :3,6-dianhydrohexitol sont des groupes comprenant de 1 à 24 atomes de carbone, de préférence des groupes comprenant de 6 à 12 atomes de carbone ;
- les esters de l'acide polycarboxylique cyclohexane ;
- les esters de l'acide phtalique.

Les composés (B) doivent être choisis pour avoir une masse molaire supérieure à 345 g.mol⁻¹. La masse molaire du composé (B) peut aller par exemple de 350 à 1000 g.mol⁻¹, préférentiellement de 390 à 600 g.mol⁻¹.

La composition selon l'invention comprend au moins un composé (B), c'est-à-dire qu'elle peut comprendre un mélange de composés (B) utiles à l'invention.

Avantageusement, le composé (B) est un diester de 1,4 : 3,6-dianhydrohexitol ou un ester de l'acide polycarboxylique cyclohexane.

Selon la variante préférée où le composé (B) est un diester de 1,4 : 3,6-dianhydrohexitol, l'ester de 1,4 : 3,6-dianhydrohexitol (B) est tout préférentiellement un ester d'isosorbide.

Préférentiellement, le groupe ester de l'ester (B) est un groupe alkyle, c'est-à-dire que l'ester est obtenu par réaction de 1,4 :3,6-dianhydrohexitol avec un ou plusieurs monoacides carboxyliques saturés. Le groupe alkyle peut être un groupe cycloalkyle, alkyle linéaire ou alkyle ramifié. Préférentiellement, le groupe alkyle est linéaire ou ramifié, tout préférentiellement linéaire.

Le ou les groupes esters de l'ester de 1,4:3,6-dianhydrohexitol comprennent de 1 à 24 atomes de carbone, c'est-à-dire que l'acide réagissant avec le 1,4 : 3,6-dianhydrohexitol comprend de 1 à 24 atomes de carbone. Avantageusement, le ou les groupes esters comprennent de 4 à 16 atomes de carbone, de manière préférée comprennent de 5 à 11, par exemple de 6 à 12, tout préférentiellement de 7 à 10.

Cet ester (B) peut être choisi parmi les diheptanoate de 1,4 : 3,6-dianhydrohexitol, les dioctanoate de 1,4 : 3,6-dianhydrohexitol, les dinonanoate de 1,4 : 3,6-dianhydrohexitol, les didécanoate de 1,4 : 3,6-dianhydrohexitol, les diundécanoate de 1,4 : 3,6-dianhydrohexitol, les didodécanoate de 1,4 : 3,6-dianhydrohexitol, les heptanoate hexanoate de 1,4 : 3,6-dianhydrohexitol, le heptanoate octanoate de 1,4 : 3,6-dianhydrohexitol, les heptanoate nonanoate de 1,4 : 3,6-dianhydrohexitol, les heptanoate décanoate de 1,4 : 3,6-dianhydrohexitol, les heptanoate undécanoate de 1,4 : 3,6-dianhydrohexitol, les heptanoate dodécanoate de 1,4 : 3,6-dianhydrohexitol, les octanoate valérate de 1,4 : 3,6-dianhydrohexitol, les octanoate isovalérate de 1,4 : 3,6-dianhydrohexitol, les octanoate hexanoate de 1,4 : 3,6-dianhydrohexitol, les octanoate nonanoate de 1,4 : 3,6-dianhydrohexitol, les octanoate décanoate de 1,4 : 3,6-dianhydrohexitol, les octanoate undécanoate de 1,4 : 3,6-dianhydrohexitol, les octanoate dodécanoate de 1,4 : 3,6-dianhydrohexitol, les nonanoate butyrate de 1,4 : 3,6-dianhydrohexitol, les nonanoate valérate de 1,4 : 3,6-dianhydrohexitol, les nonanoate isovalérate de 1,4 : 3,6-dianhydrohexitol, les nonanoate hexanoate de 1,4 : 3,6-dianhydrohexitol, les nonanoate décanoate de 1,4 : 3,6-dianhydrohexitol, les nonanoate undécanoate de 1,4 : 3,6-dianhydrohexitol, les nonanoate dodécanoate de 1,4 : 3,6-dianhydrohexitol, les décanoate butyrate de 1,4 : 3,6-dianhydrohexitol, les décanoate valérate de 1,4 : 3,6-dianhydrohexitol, les décanoate isovalérate de 1,4 : 3,6-dianhydrohexitol, les décanoate hexanoate de 1,4 : 3,6-dianhydrohexitol, les décanoate undécanoate de 1,4 : 3,6-dianhydrohexitol, les décanoate dodécanoate de 1,4 : 3,6-dianhydrohexitol, les undécanoate butyrate de 1,4 : 3,6-dianhydrohexitol, les undécanoate valérate de 1,4 : 3,6-dianhydrohexitol, les undécanoate isovalérate de 1,4 : 3,6-dianhydrohexitol, les undécanoate hexanoate de 1,4 : 3,6-dianhydrohexitol et les undécanoate dodécanoate de 1,4 : 3,6-dianhydrohexitol,.

Comme pour l'ester (A), cet ester (B) est susceptible d'être produit par réaction d'estérification du 1,4:3,6-dianhydrohexitol avec un acide carboxylique ou un mélange de ces acides.

A titre d'exemple d'acide carboxylique, on peut citer l'acide butyrique, l'acide isobutyrique, l'acide valérique, l'acide isovalérique l'acide hexanoïque, l'acide heptanoïque, l'acide octanoïque, l'acide octanoïque, l'acide nonanoïque, l'acide décanoïque, l'acide undécanoïque, l'acide dodécanoïque, l'acide tridécylique, l'acide myristique, l'acide pentadécylique, l'acide palmitique.

Ces composés (B) peuvent être obtenus en réalisant une réaction d'estérification entre le 1,4 :3,6-dianhydrohexitol avec un ou différents acides carboxyliques. Dans le cas où une seule des deux fonctions alcool du diol a réagi par estérification, l'ester est un monoester. Il s'agit d'un diester dans le cas où les deux fonctions alcool du diol ont réagi dans une réaction d'estérification. L'ester de 1,4:3,6-dianhydrohexitol est préférentiellement un diester. Selon ce mode de réalisation, le diester de 1,4:3,6-dianhydrohexitol peut comprendre différents groupes esters, c'est-à-dire que le diester est obtenu avec 2 acides différents.

La synthèse des esters de 1,4 :3,6-dianhydrohexitol est également décrite dans le document WO 99/45060 déjà cité.

Les esters de l'acide polycarboxylique cyclohexane sont connus et sont décrits par exemple dans la demande WO 99/32427 ou encore la demande WO 00/78853. A la condition que leur masse molaire dépasse 345 g.mol⁻¹, les esters de l'acide polycarboxylique cyclohexane cités de la page 5 ligne 14 à la page 12 ligne 9 de la demande WO 00/78853 peuvent être utilisés comme composé (B).

On préfère utiliser les esters d'acide dicarboxylique cyclohexane et en particulier les diesters de cet acide. On utilise avantageusement les esters d'acide dicarboxylique 1,2-cyclohexane. De préférence, les esters d'acide polycarboxylique cyclohexane sont des esters de diisonoyle, comme par exemple l'ester diisosonylique d'acide dicarboxylique 1,2-cyclohexane. Ce dernier est commercialisé par BASF sous la marque Hexamoll®. On peut obtenir ces composés de manière connue en faisant réagir de l'acide polycarboxylique cyclohexane avec un alcool ou par hydrogénation de phtalates.

Les esters de l'acide phtalique (ou phtalates) sont également des plastifiants bien connus. A titre d'exemple, il peut s'agir de dioctylphtalate ou de diisononyle phtalate. On peut obtenir ces composés en faisant réagir de l'acide phtalique avec un alcool.

Il est précisé que le ou les acides carboxyliques réagissant avec le 1,4 :3,6-dianhydrohexitol sont choisis afin que le composé (B) présente une masse molaire supérieure à 345 g.mol⁻¹. De même, il est précisé que le ou les alcools réagissant avec l'acide polycarboxylique cyclohexane ou l'acide phtalique sont choisis afin que le composé ester (B) présente une masse molaire supérieure à 345 g.mol⁻¹.

Avantageusement, la composition selon l'invention comprend, par rapport à la masse totale de la composition, au moins 50% de (A) et (B), préférentiellement au moins 80%, plus préférentiellement au moins 90%, et encore plus préférentiellement au moins 95%. La composition selon l'invention est de manière très avantageuse constituée de (A) et (B).

Selon le procédé et la composition de l'invention, la quantité massique de (A) va avantageusement de 0,1 à 99% par rapport à la masse totale de (A) et (B), avantageusement de 0,5 à 50%.

Les avantages du procédé et de la composition selon l'invention sont particulièrement marqués lorsque la quantité massique de (A) est comprise dans la gamme allant de 1 à 25% par rapport à la masse totale de (A) et (B), avantageusement allant de 2 à 20%, par exemple allant de 4 à 19%.

La composition selon l'invention, qui est une composition plastifiante apte à plastifier des polymères de manière rapide, a en plus l'avantage de présenter une très bonne tenue au froid.

Un autre objet de l'invention porte sur l'utilisation de la composition selon l'invention pour plastifier un polymère (C).

L'invention a également pour objet un procédé pour plastifier un polymère, caractérisé en ce qu'il comprend une étape consistant à mélanger ledit polymère avec la composition selon l'invention.

L'invention a également pour objet un procédé pour diminuer la température de transition vitreuse d'un polymère, caractérisé en ce qu'il comprend une étape consistant à mélanger ledit polymère avec la composition selon l'invention.

Le mélange est réalisé de préférence à l'aide d'une énergie thermomécanique de manière à mélanger intimement la composition et le polymère.

Le polymère à plastifier (C) peut être choisi parmi les polymères vinyliques tels que le polychlorure de vinyle, les polyuréthanes, les polyesters, les polymères cellulosiques, les amidons, les polymères acryliques, les polyacétates et les polyamides et les mélanges de ces polymères. Avantageusement, il s'agit de polymère vinylique, préférentiellement de polychlorure de vinyle. Par polychlorure de vinyle, on entend selon la présente invention les homopolymères de chlorure de vinyle ou les copolymères comprenant du chlorure de vinyle, par exemple les copolymères acétate de vinyle/chlorure de vinyle.

Le polymère ainsi obtenu est un polymère plastifié par la composition contenant (A) et (B). Pour l'homme du métier, cela signifie que le polymère (C) et la composition contenant (A) et (B) sont intimement mélangés. Les constituants (A) et (B), qui sont naturellement à l'état liquide, sont introduits entre les chaînes du polymère solide et il en résulte un polymère plastifié constitué d'une phase solide.

Préalablement à son mélange avec (A) et (B), le polymère (C) peut être sous toute forme, par exemple sous forme de granulés ou de poudre.

Un autre objet de l'invention porte sur une pâte de polymère comprenant un mélange d'une poudre de polymère et de la composition selon l'invention. Cette pâte est généralement appelée *plastisol* et permet de former des objets par les procédés décrits ci-après. Préférentiellement, le diamètre moyen de particules de la poudre est compris entre 1 et 30 µm, par exemple entre 1 et 20 µm. Dans le cas du polychlorure de vinyle, on peut obtenir ce type de poudres en préparant le PVC par émulsion ou micro-suspension. Cette pâte est généralement obtenue par mélange mécanique, préférentiellement sans chauffage, de la poudre de polymère (C) avec (A) et (B).

Pendant le mélangeage, le polymère (C) se délite et le diamètre moyen des particules de polymère diminue. Dans le plastisol, les particules ont généralement un diamètre moyen d'environ 0,5 à 30 µm, par exemple de 0,5 à 20 µm. Les mélanges ainsi obtenus sont appelés plastisols qui sont, selon les quantités de (A), (B) et (C), plus ou moins fluides. Classiquement, les plastisols sont préparés dans des mélangeurs rapides de type turbine, des mélangeurs planétaires ou des mélangeurs lents qui sont des mélangeurs planétaires à pales horizontales en Z.

Les constituants (A), (B) et (C) sont avantageusement dans des proportions massiques telles que la somme de (A) et (B) va de 1 à 900 parts pour 100 parts de polymère (C), avantageusement de 5 à 150 parts, préférentiellement de 10 à 120 parts de (A) et (B). On peut les introduire dans le système mélangeur par tout moyen adapté, telle que trémie d'alimentation, ou de façon manuelle.

Dans le cas de la pâte de polymère, on préfère que les quantités de plastifiant aillent de 30 à 80 parts de (A) et (B) pour 100 parts de poudre de polymère.

Dans la composition de polymère plastifié, on peut également utiliser en plus des constituants (A), (B) et (C), des additifs optionnels. Ces additifs peuvent être choisis parmi les stabilisants, les anti-UV, les charges, les colorants, les pigments, les agents gonflants, les émulsifiants, les abaisseurs de viscosité différents de (A), les épaississants, les agents de démoulage, les agents matant, les agents d'adhésion, les agents antistatique, les agents fongicides et les agents odoriférant. Les quantités de chaque additif sont choisies afin d'apporter les propriétés désirées lors de la mise en oeuvre du procédé ou pour l'objet finalement obtenu. Ces additifs peuvent être introduits dans la composition directement ou en mélange La quantité en additif optionnel va généralement de 1 à 600 parts pour 100 parts de polymère (C), généralement de 2 à 80 parts.

On peut, toujours selon l'invention, fabriquer des objets comprenant la composition de polymère plastifié en utilisant un procédé de fabrication d'un objet à base d'une composition de polymère plastifié contenant un polymère (C) et les constituants (A) et (B) tels qu'utilisés dans la composition de l'invention. Ce procédé comprend :
- une étape de sélection de l'ester (A), du composé (B) et du polymère (C) ;
- une étape d'introduction des constituants (A), (B) et (C) dans un système mélangeur, les quantités de (A) et (B) étant introduites dans les proportions telles que définies dans la composition de l'invention précédemment décrite ;
- une étape de mélange des constituants (A), (B) et (C) ;
- une étape de chauffage de ce mélange ;
- une étape de mise en forme du mélange sous la forme d'un objet ;
- enfin une étape de récupération dudit objet comprenant la composition de polymère plastifié ;
l'étape d'introduction des constituants (A), (B) et (C) dans le mélangeur pouvant se faire séparément ou par l'intermédiaire d'un mélange de constituants, et de manière simultanée ou séquencée ; et
les étapes de mélange et de chauffage pouvant être réalisées de manière simultanée ou de manière séquencée.

Avantageusement, on introduit les constituants (A) et (B) dans le système mélangeur par l'intermédiaire de la composition selon l'invention.

Selon une première variante du procédé de l'invention, le procédé comprend une étape de mélange thermomécanique.

Selon cette première variante, l'étape de mélange thermomécanique est réalisée dans un système mélangeur qui est un mélangeur pour thermoplastiques. Ce mélangeur peut être choisi parmi les malaxeurs, les mélangeurs BUSS, les mélangeurs à cylindres et les extrudeuses.

Les constituants (A) et (B) peuvent être introduits sous la forme d'un ou plusieurs mélanges-maîtres.

L'étape de mélange thermomécanique est réalisée à une température adaptée à la température de transformation du polymère (C). A titre d'exemple, la température du mélange lors du mélange thermomécanique est préférentiellement comprise entre 60 et 200°C pour un PVC.

Pour un mélange thermomécanique, on peut utiliser un polymère sous tout type de forme.

Selon cette première variante, on réalise avantageusement une étape préalable de mélange à sec (en anglais *dry blend*) des constituants (A), (B) et (C) avant le mélange thermomécanique. Ce mélange à sec peut être réalisé dans un simple mélangeur mécanique.

Selon cette variante, le procédé selon l'invention est particulièrement intéressant lorsque l'étape de mise en forme est une étape de calandrage. En effet, le calandrage est réalisé dans une calandreuse, qui est un système ouvert. Or, le procédé est particulièrement avantageux dans ce cas, car les quantités de COV émises lors du procédé sont particulièrement faibles par rapport à celles émises dans les procédés utilisant d'autres accélérateurs de gélification que les composés (A).

L'objet peut également avantageusement être mis en forme par d'autres méthodes, notamment par injection, extrusion injection, moulage, extrusion moulage, thermoformage, extrusion formage, extrusion gainage, extrusion soufflage. On peut également utiliser des techniques de co-extrusion pour former des objets multicouches.

Selon une seconde variante, on utilise pour former l'objet selon l'invention un procédé de type *plastisol* avec la pâte de polymère préalablement décrite.

Dans ce type de procédé, l'étape de mise en forme est une généralement une étape d'enduction, de trempage, d'embouage ou de moulage par rotation de la pâte de polymère, ce qui permet de former un objet préformé.

L'étape de chauffage du procédé est une étape de cuisson dudit objet préformé, qui peut avoir lieu pendant l'étape de mise en forme de l'objet préformé (c'est le cas par exemple du trempage, de l'embouage ou du moulage par rotation) ou avoir lieu après l'étape de mise en forme de l'objet préformé (c'est le cas par exemple de l'enduction). Cette étape de cuisson peut se faire à une température comprise entre 60 et 300°C, par exemple entre 100 et 250°C. Elle peut se faire sous air ou sous atmosphère contrôlée, par exemple sous atmosphère inerte.

L'étape de mise en forme de l'objet est préférentiellement une étape d'enduction de la pâte de polymère sur un support, cette enduction étant réalisée avant l'étape de cuisson dudit support enduit. L'étape d'enduction peut être réalisée sur un support textile, un polymère synthétique ou un papier.

L'enduction peut être réalisée à l'aide de toute tête d'enduction, par exemple à l'aide d'une racle ou d'un cylindre.

Cette enduction peut être, selon une première sous-variante, une enduction dite « enduction sur support » comme décrite ci-dessus ou selon une seconde sous-variante, une enduction dite « enduction sans support ». Dans ce dernier cas, le support du support enduit peut être détaché après cuisson et le procédé comprend en outre une étape ultérieure de séparation du support pour former un film ou une feuille de polymère plastifié. Un tel support peut être en papier siliconé.

L'étape de cuisson est généralement réalisée dans un four, par exemple un four tunnel.

Un autre objet de l'invention porte sur un objet comprenant la composition de polymère plastifié, qui est susceptible d'être obtenu par le procédé de l'invention.

L'objet comprenant la composition de polymère plastifié peut être tout type d'objet, tel qu'un film, une feuille, un granulé, un revêtement de sol, un revêtement mural, un tissu enduit plastique, notamment un cuir artificiel, par exemple pour chaussure, pour maroquinerie ou pour ameublement, une bâche, un *liner* par exemple pour piscine, un store, un container souple, un vêtement, un produit médical, un gant, une botte, un joint, un revêtement protecteur, un mannequin pour vitrine, un jouet par exemple un ballon ou une poupée, un tube, des profilés notamment des profilés de fenêtre, des pièces automobiles telles que tableau de bord, siège, réservoir ou appuie-tête. Ces pièces peuvent être des pièces moussées ou meringuées, c'est-à-dire comprenant des cellules d'air. Elles peuvent également être au contraire des pièces massiques.

De plus, la Demanderesse a également découvert que certains composés esters de 1,4 :3,6 dianhydrohexitol avaient la capacité d'accélérer la plastification ou la gélification de polymères, ceci en émettant des faibles quantités de COV lors du procédé de mise en oeuvre.

L'invention porte ainsi également sur l'utilisation des esters (A) décrits précédemment comme accélérateurs de gélification dans un procédé de mise en forme de polymère. Généralement, on considère qu'un composé est un accélérateur de gélification lorsqu'il a la capacité à fondre le polymère plus rapidement qu'un plastifiant de type diisononyle phtalate (DINP). Une manière simple de le mesurer est de former un premier mélange à partir de 60 parts de composé et 100 parts d'une poudre de polymère et de mesurer le développement de la viscosité de ce mélange en fonction de la température à l'aide d'un rhéomètre plan-plan. Pour mesurer cette évolution de la viscosité, on peut déposer ce mélange dans un rhéomètre, et appliquer une vitesse de cisaillement de 10 s⁻¹ avec un angle de rotation de 2°. Avant la mesure, le mélange est conditionné pendant 10 minutes en appliquant le taux de cisaillement mentionné ci-dessus et ensuite on augmente la température dans le rhéomètre, par exemple à une vitesse de 5,7 K/min et on mesure la viscosité de ce premier mélange jusqu'à ce que le polymère fonde et sa viscosité atteigne 10 Pa.s⁻¹. On reproduit ce test dans lequel on utilise un second mélange identique au premier mélange, à la différence près que le DINP remplace le composé à tester. Si le premier mélange comprenant le composé à tester atteint une viscosité de 10 Pa.s⁻¹ à une température plus faible que le second mélange, alors le composé testé est un accélérateur de gélification.

Après formation de l'objet, un autre avantage de ces accélérateurs est qu'on observe une exsudation faible lors de l'usage de l'objet en comparaison avec des objets comprenant les accélérateurs de gélification déjà connus.

L'invention a également pour objet un procédé pour accélérer la gélification d'un mélange comprenant un polymère et un plastifiant, caractérisé en ce que l'on ajoute audit mélange un ester de 1,4 : 3.6-dianhydrohexitol (A) selon l'invention dont la masse molaire va de 255 à 345 g.mol⁻¹, choisi parmi les monoesters et les diesters d'isosorbide, d'isomannide et d'isoidide.

Des modes de réalisations particuliers de l'invention vont maintenant être décrits en détail dans les exemples qui suivent. Il est précisé que ces exemples particuliers ne limitent en rien la présente invention.

### Exemples

### Exemple 1 : Propriétés de volatilité des accélérateurs de gélification

Les accélérateurs de gélification utilisés dans l'exemple qui suit sont :
- DVI (Accélérateur de gélification selon l'invention) : Di-valérate d'isosorbide présentant une masse molaire de 314 g.mol⁻¹;
- DBP : phtalate de dibutyle ;
- JAYFLEX™ MB 10 : monobenzoate d'isodécyle (Exxon Mobil) ;
- SANTICIZER® 9500 : monobenzoate de 2-ethyl hexyle (Ferro) ;

### Préparation et essais :

La détermination de la volatilité des accélérateurs de gélification est effectuée par différence de pesée après un temps de résidence défini dans une étuve ventilée. On pèse précisément dans un cristallisoir 5 g du produit à tester. Le cristallisoir est ensuite placé à l'intérieur de l'étuve à 180 °C, pendant 30 mn. Une fois ce temps écoulé, le cristallisoir est placé dans un dessiccateur jusqu'à refroidissement. Le cristallisoir est ensuite pesé et on calcule alors la volatilité selon la formule suivante : (masse plastifiant départ - masse plastifiant après séjour en étuve) x 100 / masse plastifiant départ.

Dans le Tableau 1 figurent les résultats obtenus sur les produits testés.

**Tableau 1**

| Plastifiants | Volatilité en % |
|---|---|
| DVI (accélérateur selon l'invention) | 11 |
| SANTICIZER® 9500 | 93 |
| DBP | 24 |
| JAYFLEX™ MB 10 | 40 |

Les essais montrent que le DVI se différencie nettement des autres accélérateurs de gélification par une volatilité beaucoup moins importante. Ainsi, il n'a pas le niveau de volatilité que l'on rencontre habituellement pour ces composés de faible masse molaire. Il aura donc, par ailleurs, pour effet de dégager moins de COV lors du procédé de mise en forme ainsi que lors de son utilisation.

La Demanderesse a pu constater avec surprise que, malgré cette très faible volatilité, le produit possède des propriétés d'accélération de gélification des polymères excellentes comme le montre l'exemple 2 qui suit.

### Exemple 2 : Propriétés d'accélération de la gélification de polymères

Les accélérateurs de gélification utilisés sont :
- DVI (accélération de gélification selon l'invention) : Di-valérate d'isosorbide
- CITROFOL® B2 : Acétyl TriButyl Citrate (Jungbunzlauer)
- JAYFLEX™ MB 10 : monobenzoate d'isodécyle (Exxon Mobil)
- DHP : diester phtalique d'alcool saturé comprenant 7 atomes de carbone (accélérateur utilisé dans le document US 2007/0027242) ;
Les formulations de plastisols sont réalisées à l'aide des produits suivants :
- SOLVIN® 372 NF : PVC Emulsion ; 100 parts
- Accélérateur de gélification : 60 parts
- LANKROMARK® LZB 753 : Stabilisant thermique à base de Ba/Zn ; 2 parts

Préparation des pâtes de PVC :

### 2.1 Evaluation des accélérateurs de gélifications

L'accélérateur de gélification est introduit dans un récipient en plastique contenant le PVC en même temps que le stabilisant thermique. La préparation est ensuite agitée à l'aide d'un moteur muni d'une pale d'agitation de type RAYNERI, à vitesse lente. Puis, la vitesse de mélange est augmentée à 2000 t/min pendant 150 secondes. La préparation est ensuite placée dans un dessiccateur sous vide afin d'en ôter les bulles d'air. La pâte de PVC ainsi obtenue est également appelée « plastisol ».

La pâte est ensuite utilisée pour mesurer le développement de sa viscosité en fonction de la température à l'aide d'un rhéomètre de type Physica MCR Rheometer. Pour mesurer cette évolution de la viscosité, une goutte de la pâte est placée sur un plateau de 50 mm de diamètre et un angle de 2°. Le taux de cisaillement choisi est de 10 s⁻¹ et le gradient de température de 5,7 K/min. Avant la mesure, la pâte est conditionnée pendant 10 minutes en appliquant le taux de cisaillement mentionné ci-dessus et ensuite le gradient de température est appliqué. La mesure est stoppée lorsque la température atteint 150°C ou si le couple résultant de la mesure atteint une valeur trop élevée pour le système de mesure.

Les résultats de l'évolution de la viscosité en fonction de la température selon ce protocole de mesure sont présentés dans le Tableau 2 ci-dessous, au travers de l'expression d'un angle « Alpha », angle déterminé à partir de la tangente à la courbe d'évolution de la viscosité en fonction de la température (cf Figure 1).

La température pour laquelle la pâte de PVC a atteint une viscosité égale à 10 Pa.s (notée « T à 10 Pa.s», cf Figure 1) est aussi relevée comme un critère pertinent d'évaluation de l'efficacité des accélérateurs de gélification.

**Tableau 2**

| Accélérateur | Alpha en degrés | T à 10 Pa.s en °C |
|---|---|---|
| DVI (Accélérateur selon l'invention) | 79 | 67 |
| CITROFOL® B2 | 72 | 83 |
| JAYFLEX™ MB 10 | 71 | 76 |
| DHP | 73 | 83 |

On peut noter que plus la valeur de l'angle Alpha est élevée, plus la vitesse d'augmentation de la viscosité de la pâte de PVC est importante, et donc plus l'accélérateur est efficace. L'angle Alpha observé pour le DVI est significativement plus élevé que ceux obtenus avec les produits CITROFOL® B2 et JAYFLEX™ MB 10. Selon ces essais, le DHP est quant à lui encore moins bon accélérateur de gélification que les produits CITROFOL® B2 et JAYFLEX™ MB 10. Le DVI est donc un excellent accélérateur de gélification du PVC, plus efficace que de nombreux accélérateurs de gélification du marché.

De plus, la température nécessaire pour que la pâte de PVC atteigne une viscosité de 10 Pa.s est la plus basse avec le DVI. Ceci conforte et confirme une plus grande rapidité de l'accélérateur selon l'invention, puisque la température nécessaire à amorcer la gélification est plus basse de 9 à 16°C vis-à-vis des accélérateurs de gélification du marché testés. Ainsi, un gain d'énergie significatif pourra être réalisé lors de la gélification des plastisols en utilisant l'accélérateur de gélification selon l'invention.

### 2.2 Evaluation de compositions plastifiantes

La Demanderesse a également réalisé à l'aide de ce même protocole, des essais en utilisant des compositions plastifiantes selon l'invention et comparatives, mélangeant un plastifiant (P) avec un accélérateur de gélification (AG).

Les plastifiants (P) :
- DEI : DiEster octanoïque d'Isosorbide ayant une masse molaire de 398 g.mol⁻¹
- DINP: Di Iso Nonyl Phtalate (Sigma Aldrich)
- Hexamoll® DINCH : Di IsoNonyl CycloHexane (Basf)
Les accélérateurs de gélification (AG) :
- DVI (accélérateur utilisé dans l'invention) : Di-valérate d'isosorbide
- DBP : phtalate de dibutyle ;
- DHP : diester phtalique d'alcool saturé comprenant 7 atomes de carbone (accélérateur utilisé dans le document US 2007/0027242) ;
- JAYFLEX™ MB 10 : monobenzoate d'isodécyle (Exxon Mobil)
- SANTICIZER® 9500 : monobenzoate de 2-ethyl hexyle (Ferro)

Les formulations de plastisols sont réalisées à l'aide des produits suivants :
- SOLVIN® 372 NF : PVC Emulsion ; 100 parts
- Composition plastifiante : 60 parts avec des ratio variables de plastifiant et d'accélérateur de gélification (P/AG)
- BAEROSTAB® NT 319P : Stabilisant thermique à base de Ba/Zn, 1,5 parts
- BAEROSTAB LSA® : Co-stabilisant à base d'huile de soja époxydée, 2 parts

Le protocole de préparation des pâtes de PVC est identique à celui décrit précédemment.

On observe, comme un critère pertinent d'évaluation de l'efficacité des compositions plastifiantes, la température pour laquelle la pâte de PVC a atteint :
- une viscosité égale à 10 Pa.s (notée « T à 10 Pa.s»),
- une viscosité égale à 100 Pa.s (notée « T à 100 Pa.s», cf Figure 1).

On obtient avec des PVC comprenant du DEI et du DINCH comme seul plastifiant, les résultats reportés dans le Tableau 3 :

**Tableau 3**

| Plastifiant P | T à 10 Pa.s (°C) | T à 100 Pa.s (°C) |
|---|---|---|
| DEI | 79 | 90 |
| DINCH | 95 | 126 |

Le Tableau 4 ci-après présente les résultats obtenus pour les différentes pâtes à base de PVC et de compositions plastifiantes.

**Tableau 4**

| P | AG | T à 10 Pa.s (°C) | | T à 100 Pa.s (°C) | |
|---|---|---|---|---|---|
| | | Ratio 95/5 (P/AG) | Ratio 80/20 (P/AG) | Ratio 95/5 (P/AG) | Ratio 80/20 (P/AG) |
| DEI | DVI | 77 | 74 | 87 | 82 |
| DEI | SANTICIZER® 9500 | 78 | 75 | 90 | 83 |
| DEI | DBP | 76 | 72 | 86 | 79 |
| DEI | JAYFLEX™ MB 10 | 78 | 77 | 90 | 89 |
| DEI | DHP | 79 | 78 | 90 | 89 |
| DINCH | DVI | Non mesuré | 85 | Non mesuré | 101 |
| DINCH | JAYFLEX™ MB10 | Non mesuré | 92 | Non mesuré | 122 |
| DINCH | CITROFOL® B2 | Non mesuré | 90 | Non mesuré | 100 |
| DINCH | DHP | Non mesuré | 93 | Non mesuré | 120 |
| DINP | DVI | 80 | 76 | 89 | 83 |
| DINP | DHP | 82 | 79 | 93 | 87 |

Ainsi, en comparant ces derniers résultats avec ceux du Tableau 3, les essais montrent que, dès l'ajout de 5 parts de DVI, la température de gélification est diminuée, et ceci quel que soit le plastifiant utilisé. Cette diminution est d'autant plus significative lorsque la quantité d'accélérateur de gélification augmente.

De plus, le DVI apparait bien ici comme un accélérateur de viscosité aussi performant voire plus performant que les accélérateurs déjà connus. Il est notamment plus efficace que le DHP, et ceci quel que soit le plastifiant utilisé (DEI, DINCH ou DINP).

Le DHP ne se comporte pas comme un accélérateur de gélification lorsqu'il est utilisé dans un mélange plastifiant avec le DEI ou le DINCH, comme le montrent les températures de gélification du polymère, très proches respectivement de la température de gélification du polymère plastifié avec le DEI seul et le DINCH seul.

### Exemple 3 : Evaluation des propriétés mécaniques

Les formulations de PVC plastifiés selon l'invention et comparatives sont réalisées à l'aide des produits suivants :
PVC MARVYLAN® S7102 : 100 parts
Stabilisant BAEROSTAB® NT 319P (Ca/Zn poudre) : 1,5 part
Co-stabilisant BAEROSTAB® LSA (huile de soja époxydée) : 2 parts
Plastifiant et accélérateur de gélification : 34 parts

La préparation des éprouvettes pressées destinées à la caractérisation des propriétés mécaniques se fait en plusieurs étapes.

Dans un premier temps, il est nécessaire de plastifier du PVC poudre avec la composition plastifiante dans un mélangeur planétaire de type PLANETMIX 500 (Sté Thermo Fisher) équipé d'un polystat pour assurer la régulation de la température. On remplit ce mélangeur avec du PVC, le stabilisant thermique et le co-stabilisant thermique. On incorpore ensuite la composition plastifiante sur toute la surface de la poudre de PVC lorsque la température atteint 85 °C. La préparation est ainsi mélangée pendant 8 mn.

Dans un second temps, on réalise des plaques de PVC plastifié à l'aide d'une presse de type CARVER et d'un moule de 30 x 30 cm en inox poli miroir muni d'un cadre de 2 mm d'épaisseur et d'un couvercle inox poli miroir. Ainsi, on place le cadre à l'intérieur du moule et on y verse 180 g de poudre PVC plastifiée. La poudre est répartie uniformément et on la recouvre avec le couvercle. L'ensemble est placé sur le plateau de la presse et on programme une force de fermeture de 18 000 kg pendant 2 mn puis un refroidissement jusqu'à 40 °C à 50 °C. On démoule alors la plaque de PVC ainsi obtenue.

Enfin, dans une dernière étape, à partir des plaques de PVC plastifié obtenues comme décrit précédemment, on découpe 10 éprouvettes de type 5A à l'aide d'un emporte-pièce (dimension des éprouvettes = Longueur : 25mm ; Largeur : 4mm ; Epaisseur : 2mm).

Ces éprouvettes sont ensuite caractérisées en traction sur un banc de traction de type LLOYD Modèle « LR5K plus » avec les paramètres suivants : Vitesse d'avancement : 50 mm / min ; Cellule : 5 KN ; la précontrainte est remise à zéro une fois que l'éprouvette est en place et que les mors sont serrés.

Une fois que l'essai est terminé, on relève le module d'Young et le pourcentage d'allongement à rupture.

Le Tableau 5 ci-après présente, pour chaque composition testée l'ensemble des propriétés mécaniques modifiées par la plastification, à savoir le module de rigidité (module d'Young) et le taux d'allongement à la rupture.

**Tableau 5**

| **Plastifiant** | **Module d'Young (MPa)** | **Allongement à la rupture (%)** |
|---|---|---|
| DEI | 5,0 | 591 |
| DEI / DVI (ratio 80/20) | 4,6 | 612 |
| DEI / SANTICIZER® 9500 (ratio 80/20) | 4,6 | 568 |
| DEI / JAYFLEX™ MB10 (ratio 80/20) | 5,7 | 549 |
| DEI / DBP (ratio 80/20) | 4,9 | 589 |
| DINP seul | 6,2 | 551 |
| DINP / DVI (ratio 80/20) | 6,5 | 529 |
| DINCH seul | 6,7 | 520 |
| DINCH / DVI (ratio 80/20) | 6,7 | 490 |

Ces essais montrent que l'ajout d'accélérateur de gélification du marché tels que du DBP ou du SANTICIZER® 9500 ne modifie que faiblement les propriétés mécaniques d'un PVC plastifié avec du DEI comme plastifiant.

De la même manière, l'ajout de DVI, comme accélérateur de gélification, ne modifie pas les propriétés mécaniques des PVC plastifiés. L'ajout de DVI à un taux de 20% dans la composition plastifiante DEI/DVI, induit une très faible chute de module de 8% et une augmentation de l'allongement à rupture de 3,5% également faible. Ces résultats sont comparables à ceux obtenus avec le DBP. Le même phénomène a pu être observé avec d'autres plastifiants que le DEI (DINCH, DINP). L'ajout de DVI ne dégrade donc pas les propriétés mécaniques du PVC obtenu. Ainsi, l'utilisation de DVI permet de conserver les propriétés mécaniques des polymères plastifiés.

### Exemple 4 : Effet sur les propriétés de migration de la composition plastifiante

### Préparation des essais :

Sur une plaque de PVC telle que réalisée dans l'exemple 3, on découpe des éprouvettes de PVC (40 x 40 mm, épaisseur 2 mm). Elles sont conditionnées pendant 72 h à 20 °C - 65 % HR. On réalise de même avec des supports absorbants (buvard CANSON de 10x10cm (100 cm²)). Puis les éprouvettes et les supports absorbants sont pesés sur une balance de précision. Ensuite, les éprouvettes de PVC plastifiées sont mises entre les 2 supports absorbants, au centre de ceux-ci. Cet ensemble est disposé entre 2 plaques de verre, et on pose sur le dessus un poids de 5 kg. Le tout est placé dans une étuve ventilée à 70°C pendant 4 semaines. Au bout de 4 semaines, les éprouvettes sont mises à conditionner à nouveau à 20 °C - 65 % HR pendant 2 jours. Enfin, elles sont repesées afin de déterminer le taux de migration de l'éprouvette, comme suit : (masse éprouvette avant étuve - masse éprouvette après étuve) x 100 / masse éprouvette avant étuve.

**Tableau 6**

| **Plastifiant** | **Taux de migration (%)** |
|---|---|
| DEI | 0,29 |
| DEI / DVI (ratio 97/3) | 0,35 |
| DEI / DVI (ratio 95/5) | 0,35 |
| DEI / DVI (ratio 90/10) | 0,31 |
| DEI / DVI (ratio 85/15) | 0,30 |
| DEI / DVI (ratio 80/20) | 0,29 |

Un des critères essentiels pour tout polymère plastifié est son taux de migration de la composition plastifiante utilisée. En effet, celui-ci doit être minimal si l'on souhaite préserver les propriétés du matériau dans le temps.

Dans un ratio de 80/20, on observe un taux de migration équivalent au taux observé pour le DEI ; il n'y a donc pas, à ce ratio, de migration ajoutée par le DVI en mélange avec le DEI. Ceci est surprenant car au vu du fait qu'il se comporte comme un accélérateur de gélification, on aurait pu s'attendre à un impact important sur la migration. En effet, les taux de migration augmentent fortement lorsque l'on utilise des accélérateurs de gélification classiques du marché, comme le montre les autres résultats obtenus et regroupés dans le Tableau 7 suivant :

**Tableau 7**

| **Plastifiant** | **Taux de migration (%)** | **Pourcentage d'augmentation du taux de migration par rapport au plastifiant seul** |
|---|---|---|
| DEI seul | 0,29 | - |
| DEI / DVI (ratio 80/20) | 0,29 | 0% |
| DEI / SANTICIZER® 9500 (ratio 80/20) | 2,58 | 790 % |
| DEI / JAYFLEX™ MB10 (ratio 80/20) | 0,59 | 103 % |
| DEI / DBP (ratio 80/20) | 1,15 | 296 % |
| DEI / DHP (ratio 80/20) | 0,85 | 193 % |
| DINP seul | 0,13 | - |
| DINP / DVI (ratio 80/20) | 0,20 | 54% |
| DINCH seul | 0,19 | - |
| DINCH / DVI (ratio 80/20) | 0,22 | 16% |

Ces essais montrent que la migration ajoutée par les autres accélérateurs de gélification peut être très importante. En effet, la migration augmente de près de 300% avec DBP, de près de 200% avec le DHP, voire près de 800% avec SANTICIZER® 9500.

On a pu aussi montrer que l'accélérateur de gélification selon l'invention migre également très peu avec d'autres plastifiants que le DEI (DINP, DINCH).

Cela montre que la composition selon l'invention a la capacité, après mélange dans le polymère, de moins migrer par rapport aux compositions comparatives utilisant des accélérateurs de gélification connus, notamment ceux décrits dans le document US 2007/0027242.

### Exemple 5 : Amélioration du point de congélation à basse température du plastifiant

Des essais ont été menés afin d'évaluer l'impact de l'ajout de DVI dans le plastifiant DEI.

L'évolution de la viscosité du plastifiant en fonction de la température est mesurée à l'aide d'un rhéomètre de type Physica MCR Rheometer. Pour mesurer cette évolution de la viscosité, une goutte du plastifiant est placée sur un plateau de 50 mm de diamètre et un angle de 1 ° (géométrie CP 50-1) pour une mesure des modules visqueux et élastiques en fonction de la température. Le gradient de température est de 2°C/min, le balayage en température de 20°C à -50°C, la fréquence d'oscillation de 1 Hertz et la déformation de 1 à 0,1%. La température de congélation mesurée correspond à la température de changement d'état du produit soumis à l'essai et correspond au croisement des modules visqueux et élastiques.

Le Tableau 8 reprend les mesures de la température de congélation.

**Tableau 8**

| **Plastifiant** | **Température de congélation (°C)** |
|---|---|
| DEI | -7 |
| DEI / DVI (ratio 90/10) | -11 |
| DEI / DVI (ratio 80/20) | -14 |

L'ajout de DVI a un impact significatif sur la température de congélation du DEI.

Les plastifiants pour PVC sont des produits liquides, destinés à être stockés dans des bidons de stockages. Ces derniers peuvent être installés à l'extérieur des bâtiments de production où ils sont mis en oeuvre. Il est donc nécessaire que les plastifiants aient des points de congélation (Température à laquelle le produit commence à changer d'état pour passer de l'état liquide à celui de gel) relativement bas. Dans le cas inverse, ils doivent être isolés voire thermorégulés.

Avec l'ajout de DVI, il n'est donc plus nécessaire dans certains cas d'utiliser des systèmes de stockage isolés ou thermorégulés pour le stockage du plastifiant.

## Revendications

1. Composition comprenant par rapport à la masse totale de (A) et (B) :
- de 0,1 à 99% en masse d'au moins un ester de 1,4 : 3,6-dianhydrohexitol (A) dont la masse molaire va de 255 à 345 g.mol⁻¹ et choisi parmi les monoesters et les diesters d'isosorbide, d'isomannide et d'isoidide ;
- de 1 à 99,9% en masse d'au moins un composé (B) dont la masse molaire est supérieure à 345 g.mol⁻¹ et choisi parmi :
▪ les esters de 1,4 :3,6-dianhydrohexitol choisis parmi les monoesters et les diesters d'isosorbide, d'isomannide et d'isoidide et dont les groupes esters de 1,4 :3,6-dianhydrohexitol sont des groupes comprenant de 1 à 24 atomes de carbone, de préférence des groupes comprenant de 6 à 12 atomes de carbone;
▪ les esters de l'acide polycarboxylique cyclohexane ;
▪ les esters d'acide phtalique.

2. Composition selon la revendication 1, dans laquelle les groupes ester de l'ester de 1,4 : 3,6-dianhydrohexitol (A) sont des groupes comprenant de 2 à 8 atomes de carbone.

3. Composition selon l'une des revendications précédentes, dans laquelle le groupe ester de l'ester (A) est un groupe alkyle.

4. Composition selon la revendication précédente, dans laquelle l'ester (A) est choisi parmi les dipropionate de 1,4 : 3,6-dianhydrohexitol, les dibutyrate de 1,4 : 3,6-dianhydrohexitol, les diisobutyrate de 1,4 : 3,6-dianhydrohexitol, les divalérate de 1,4 : 3,6-dianhydrohexitol, les diisovalérate de 1,4 : 3,6-dianhydrohexitol, les dihexanoate de 1,4 : 3,6-dianhydrohexitol, les propionate butyrate de 1,4 : 3,6-dianhydrohexitol, le propionate isobutyrate de 1,4 : 3,6-dianhydrohexitol, les propionate valérate de 1,4 : 3,6-dianhydrohexitol, les propionate isovalérate de 1,4 : 3,6-dianhydrohexitol, les propionate hexanoate de 1,4 : 3,6-dianhydrohexitol, les butyrate isobutyrate de 1,4 : 3,6-dianhydrohexitol, les butyrate valérate de 1,4 : 3,6-dianhydrohexitol, les butyrate isovalérate de 1,4 : 3,6-dianhydrohexitol, le butyrate hexanoate de 1,4 : 3,6-dianhydrohexitol, les isobutyrate valérate de 1,4 : 3,6-dianhydrohexitol, les isobutyrate isovalérate de 1,4 : 3,6-dianhydrohexitol, les isobutyrate hexanoate de 1,4 : 3,6-dianhydrohexitol, les valérate hexanoate de 1,4 : 3,6-dianhydrohexitol, les isovalérate hexanoate de 1,4 : 3,6-dianhydrohexitol.

5. Composition selon la revendication précédente, dans laquelle l'ester (A) est un divalérate de 1,4 : 3,6-dianhydrohexitol ou un dihexanoate de 1,4 : 3,6-dianhydrohexitol.

6. Composition selon l'une des revendications précédentes, dans laquelle l'ester de 1,4 : 3,6-dianhydrohexitol (A) est un ester d'isosorbide.

7. Composition selon l'une des revendications précédentes, dans laquelle le composé (B) est un diester de 1,4 : 3,6-dianhydrohexitol ou un ester de l'acide polycarboxylique cyclohexane.

8. Composition selon la revendication précédente, dans laquelle le composé (B) est un diester de 1,4 : 3,6-dianhydrohexitol, préférentiellement un diester d'isosorbide.

9. Composition selon l'une des revendications 1 à 8, dans laquelle la quantité de (A) va de 0,5 à 50% de la masse totale de (A) et (B), avantageusement de 1 à 25 %, préférentiellement de 2 à 20%, par exemple de 4 à 19%.

10. Pâte de polymère comprenant un mélange d'une poudre de polymère et de la composition selon l'une des revendications 1 à 9.

11. Utilisation d'une composition selon l'une des revendications 1 à 9 pour plastifier un polymère.

12. Pâte selon la revendication 10, dans laquelle le polymère est choisi parmi les polymères vinyliques tels que le polychlorure de vinyle, les polyuréthanes, les polyesters, les polymères cellulosiques, les amidons, les polymères acryliques, les polyacétates et les polyamides ou un mélange de ces polymères, préférentiellement le polychlorure de vinyle.

13. Utilisation selon la revendication 11, dans laquelle le polymère est choisi parmi les polymères vinyliques tels que le polychlorure de vinyle, les polyuréthanes, les polyesters, les polymères cellulosiques, les amidons, les polymères acryliques, les polyacétates et les polyamides ou un mélange de ces polymères, préférentiellement le polychlorure de vinyle.

14. Procédé de fabrication d'un objet à base d'une composition de polymère plastifié, celle-ci contenant un polymère (C) et les constituants (A) et (B) tels que définis dans l'une des revendications 1 à 9, ledit procédé comprenant :
• une étape de sélection de l'ester (A), du composé (B) et du polymère (C) ;
• une étape d'introduction des constituants (A), (B) et (C) dans un système mélangeur, les quantités de (A) et (B) étant introduites dans les proportions telles que définies dans l'une des revendications 1 à 9 ;
• une étape de mélange des constituants (A), (B) et (C) ;
• une étape de chauffage de ce mélange ;
• une étape de mise en forme du mélange sous la forme d'un objet ;
• enfin une étape de récupération dudit objet comprenant une composition de polymère plastifié ;
l'étape d'introduction des constituants (A), (B) et (C) dans le mélangeur pouvant se faire séparément ou par l'intermédiaire d'un mélange de constituants, et de manière simultanée ou séquencée ;
les étapes de mélange et de chauffage pouvant être réalisées de manière simultanée ou de manière séquencée.

15. Procédé selon la revendication 14, dans lequel l'étape de mélange est une étape de mélange thermomécanique et dont le système mélangeur est un mélangeur pour thermoplastiques, préférentiellement choisi parmi les malaxeurs, les mélangeurs BUSS, les mélangeurs à cylindres et les extrudeuses.

16. Procédé selon l'une des revendications 14 ou 15, dans laquelle l'étape de mise en forme est une étape de calandrage.

17. Procédé de fabrication de l'objet selon la revendication 14, **caractérisé en ce que** :
• l'étape de mélange des constituants (A), (B) et (C) est réalisée pour former une pâte de polymère ;
• l'étape de mise en forme est une étape d'enduction, de trempage, d'embouage ou de moulage par rotation de cette pâte de polymère, afin de former un objet préformé ;
• l'étape de chauffage étant une étape de cuisson dudit objet préformé, qui peut avoir lieu pendant ou après l'étape de mise en forme, afin de former l'objet.

18. Procédé selon la revendication précédente, **caractérisé en ce que** l'étape de mise en forme est une étape d'enduction sur un support et que l'étape de chauffage est une étape de cuisson ayant lieu après l'étape d'enduction.

19. Procédé de fabrication selon l'une des revendications 14 à 18, **caractérisé en ce que** le polymère est choisi parmi les polymères vinyliques tels que le polychlorure de vinyle, les polyuréthanes, les polyesters, les polymères cellulosiques, les amidons, les polymères acryliques, les polyacétates et les polyamides et les mélanges de ces polymères, préférentiellement le polychlorure de vinyle.

20. Procédé selon l'une des revendications 14 à 19, **caractérisé en ce que** les constituants (A), (B) et (C) sont introduits dans le système mélangeur dans des proportions massiques telles que la somme de (A) et (B) va de 1 à 900 parts pour 100 parts de polymère (C), préférentiellement de 10 à 120 parts de (A) et (B).

21. Utilisation comme accélérateur de gélification de polymère d'un ester de 1,4: 3,6-dianhydrohexitol (A) dont la masse molaire va de 255 à 345 g.mol⁻¹, choisi parmi les monoesters et les diesters d'isosorbide, d'isomannide et d'isoidide.

## Patentansprüche

1. Zusammensetzung, umfassend, bezogen auf die Gesamtmasse von (A) und (B):
- 0,1 bis 99 Gew.-% wenigstens eines Esters von 1,4:3,6-Dianhydrohexitol (A), dessen molare Masse im Bereich von 255 bis 345 g.mol⁻¹ liegt und der gewählt ist aus Isosorbid-, Isomannid- und Isoidid-Monoestern und -Diestern;
- 1 bis 99,9 Gew.-% wenigstens einer Verbindung (B), deren molare Masse größer als 345 g.mol⁻¹ ist und gewählt ist aus:
▪ 1,4:3,6-Dianhydrohexitolester, gewählt aus Isosorbid-, Isomannid- und Isoidid-Monoestern und -Diestern, und deren 1,4:3,6-Dianhydrohexitolestergruppen Gruppen mit 1 bis 24 Kohlenstoffatomen, vorzugsweise Gruppen mit 6 bis 12 Kohlenstoffatomen umfassen;
▪ Ester von Polycarbonsäurecyclohexan;
▪ Phthalsäureester.

2. Zusammensetzung nach Anspruch 1, wobei die Estergruppen des 1,4:3,6-Dianhydrohexitolester (A) Gruppen mit 2 bis 8 Kohlenstoffatomen umfassen.

3. Zusammensetzung nach einem der vorhergehenden Ansprüche, wobei die Estergruppe des Esters (A) eine Alkylgruppe ist.

4. Zusammensetzung nach dem vorhergehenden Anspruch, wobei der Ester (A) gewählt ist aus 1,4:3,6-Dianhydrohexitol-Dipropionat, 1,4:3,6-Dianhydrohexitol-Dibutyrat, 1,4:3,6-Dianhydrohexitol-Diisobutyrat, 1,4:3,6-Dianhydrohexitol-Divalerat, 1,4:3,6-Dianhydrohexitol-Diisovalerat, 1,4:3,6-Dianhydrohexitol-Dihexanoat, 1,4:3,6-Dianhydrohexitol-Propionatbutyrat, 1,4:3,6-Dianhydrohexitol-Propionat-Isobutyrat, 1,4:3,6-Dianhydrohexitol- Propionat-Valerat, 1,4 :3,6-Dianhydrohexitol-Propionat-Isovalerat, 1,4:3,6-Dianhydrohexitol-Propionat-Hexanoat, 1,4:3,6-Dianhydrohexitol-Butyrat-Isobutyrat, 1,4:3,6-Dianhydrohexitol-Butyrat-Valerat, 1,4:3,6-Dianhydrohexitol-Butyrat-Isovalerat, 1,4:3,6-Dianhydrohexitol-Butyrat-Hexanoat, 1,4:3,6-Dianhydrohexitol-Isobutyrat-Valerat, 1,4:3,6-Dianhydrohexitol-Isobutyrat-Isovalerat, 1,4:3,6-Dianhydrohexitol-Isobutyrat-Hexanoat, 1,4:3,6-Dianhydrohexitol-Valerat-Hexanoat, 1,4:3,6-Dianhydrohexitol-Isovalerat-Hexanoat.

5. Zusammensetzung nach dem vorhergehenden Anspruch, wobei der Ester (A) ein 1,4:3,6-Dianhydrohexitol-Divalerat oder ein 1,4:3,6-Dianhydrohexitol-Dihexanoat ist.

6. Zusammensetzung nach einem der vorhergehenden Ansprüche, wobei der 1,4:3,6-Dianhydrohexitol-Ester (A) ein Isosorbidester ist.

7. Zusammensetzung nach einem der vorhergehenden Ansprüche, wobei die Verbindung (B) ein Diester von 1,4:3,6-Dianhydrohexitol oder ein Ester der Cyclohexan-Polycarbonsäure ist.

8. Zusammensetzung nach dem vorhergehenden Anspruch, wobei die Verbindung (B) ein 1,4:3,6-Dianhydrohexitol-Diester, vorzugsweise ein Isosorbiddiester ist.

9. Zusammensetzung nach einem der Ansprüche 1 bis 8, wobei die Menge (A) im Bereich von 0,5 bis 50% der Gesamtmasse von (A) und (B) liegt, vorteilhafterweise im Bereich von 1 bis 25%, vorzugsweise im Bereich von 2 bis 20%, beispielsweise im Bereich von 4 bis 19%.

10. Polymerpaste, umfassend eine Mischung aus einem Polymerpulver und der Zusammensetzung nach einem der Ansprüche 1 bis 9.

11. Verwendung einer Zusammensetzung nach einem der Ansprüche 1 bis 9 zum Plastifizieren eines Polymers.

12. Paste nach Anspruch 10, wobei das Polymer gewählt ist aus Vinylpolymeren wie Polyvinylchlorid, Polyurethanen, Polyestern, Cellulosepolymeren, Stärken, Acrylpolymeren, Polyacetaten und Polyamiden oder einer Mischung dieser Polymere, vorzugsweise Polyvinylchlorid.

13. Verwendung nach Anspruch 11, wobei das Polymer gewählt ist aus Vinylpolymeren wie Polyvinylchlorid, Polyurethanen, Polyestern, Cellulosepolymeren, Stärken, Acrylpolymeren, Polyacetaten und Polyamiden oder einer Mischung dieser Polymere, vorzugsweise Polyvinylchlorid.

14. Verfahren zur Herstellung eines Gegenstandes auf der Grundlage einer plastifizierten Polymerzusammensetzung, wobei letztere ein Polymer (C) und die Komponenten (A) und (B) nach einem der Ansprüche 1 bis 9 enthält, wobei das Verfahren umfasst:
• einen Schritt des Auswählens des Esters (A), der Verbindung (B) und des Polymers (C);
• einen Schritt des Einbringens der Komponenten (A), (B) und (C) in ein Mischsystem, wobei die Mengen von (A) und (B) in den in einem der Ansprüche 1 bis 9 definierten Verhältnissen eingeführt werden;
• einen Schritt des Mischens der Komponenten (A), (B) und (C);
• einen Schritt des Erwärmens dieser Mischung;
• einen Schritt des Formens der Mischung in Form eines Objekts;
• schließlich einen Schritt des Erhaltens des Objekts, welches eine plastifizierte Polymerzusammensetzung umfasst;
wobei der Schritt des Einbringens der Komponenten (A), (B) und (C) in den Mischer separat oder mittels einer Mischung von Komponenten erfolgen kann sowie gleichzeitig oder nacheinander;
wobei die Schritte des Mischens und Erwärmens gleichzeitig oder nacheinander durchgeführt werden können.

15. Verfahren nach Anspruch 14, wobei der Mischschritt ein thermomechanischer Mischschritt ist und dessen Mischsystem ein Mischer für Thermoplaste ist, vorzugsweise gewählt aus Rührwerken, BUSS-Mischern, Mischwalzwerken und Extrudern.

16. Verfahren nach einem der Ansprüche 14 oder 15, wobei der Formschritt ein Kalandrierschritt ist.

17. Verfahren zur Herstellung des Objekts nach Anspruch 14, **dadurch gekennzeichnet, dass**:
• der Schritt des Mischens der Komponenten (A), (B) und (C) durchgeführt wird, um eine Polymerpaste zu bilden;
• der Formschritt ein Schritt des Beschichtens, Tauchens, Slush-Mouldings oder Rotationsformens dieser Polymerpaste zum Formen vorgeformten Gegenstands ist;
• der Erwärmungsschritt ein Schritt es Brennens des vorgeformten Objekts ist, der während oder nach dem Formschritt stattfinden kann, um das Objekt zu formen.

18. Verfahren nach dem vorhergehenden Anspruch, **dadurch gekennzeichnet, dass** der Formschritt ein Beschichtungsschritt auf einem Träger ist und dass der Erwärmungsschritt ein Brennschritt ist, der nach dem Beschichtungsschritt erfolgt.

19. Herstellungsverfahren nach einem der Ansprüche 14 bis 18, **dadurch gekennzeichnet, dass** das Polymer gewählt ist aus Vinylpolymeren wie Polyvinylchlorid, Polyurethanen, Polyestern, Cellulosepolymeren, Stärken, Acrylpolymeren, Polyacetaten und Polyamiden und deren Mischungen, vorzugsweise Polyvinylchlorid.

20. Verfahren nach einem der Ansprüche 14 bis 19, **dadurch gekennzeichnet, dass** die Komponenten (A), (B) und (C) in solchen Massenverhältnissen in das Mischsystem eingebracht werden, dass die Summe von (A) und (B) im Bereich von 1 bis 900 Teilen pro 100 Teile Polymer (C), vorzugsweise 10 bis 120 Teilen von (A) und (B) liegt.

21. Verwendung eines 1,4:3,6-Dianhydrohexitolesters (A) mit einem Molekulargewicht von 255 bis 345 g.mol⁻¹, gewählt aus Isosorbid-, Isomannid- und Isoidid-Monoestern und -Diestern, als Polymer-Gelierungsbeschleuniger.

## Claims

1. A composition comprising, with respect to the total weight of (A) and (B):
• from 0.1% to 99% by weight of at least one 1,4:3,6-dianhydrohexitol ester (A), the molar mass of which ranges from 255 to 345 g.mol⁻¹, which is chosen from isosorbide, isomannide and isoidide monoesters and diesters;
• from 1% to 99.9% by weight of at least one compound (B), the molar mass of which is greater than 345 g.mol⁻¹, chosen from:
• 1,4:3,6-dianhydrohexitol esters chosen from isosorbide, isomannide and isoidide monoesters and diesters, the 1,4:3,6-dianhydrohexitol ester groups of which are groups comprising from 1 to 24 carbon atoms, preferably groups comprising 6 to 12 carbon atoms;
• esters of cyclohexanepolycarboxylic acid;
• esters of phthalic acid.

2. The composition as claimed in claim 1, in which the ester groups of the 1,4:3,6-dianhydrohexitol ester (A) are groups comprising from 2 to 8 carbon atoms.

3. The composition as claimed in either of the preceding claims, in which the ester group of the ester (A) is an alkyl group.

4. The composition as claimed in the preceding claim, in which the ester (A) is chosen from 1,4:3,6-dianhydrohexitol dipropionates, 1,4:3,6-dianhydrohexitol dibutyrates, 1,4:3,6-dianhydrohexitol diisobutyrates, 1,4:3,6-dianhydrohexitol divalerates, 1,4:3,6-dianhydrohexitol diisovalerates, 1,4:3,6-dianhydrohexitol dihexanoates, 1,4:3,6-dianhydrohexitol propionates butyrates, 1,4:3,6-dianhydrohexitol propionates isobutyrates, 1,4:3,6-dianhydrohexitol propionates valerates, 1,4:3,6-dianhydrohexitol propionates isovalerates, 1,4:3,6-dianhydrohexitol propionates hexanoates, 1,4:3,6-dianhydrohexitol butyrates isobutyrates, 1,4:3,6-dianhydrohexitol butyrates valerates, 1,4:3,6-dianhydrohexitol butyrates isovalerates, 1,4:3,6-dianhydrohexitol butyrates hexanoates, 1,4:3,6-dianhydrohexitol isobutyrates valerates, 1,4:3,6-dianhydrohexitol isobutyrates isovalerates, 1,4:3,6-dianhydrohexitol isobutyrates hexanoates, 1,4:3,6-dianhydrohexitol valerates hexanoates and 1,4:3,6-dianhydrohexitol isovalerates hexanoates.

5. The composition as claimed in the preceding claim, in which the ester (A) is a 1,4:3,6-dianhydrohexitol divalerate or a 1,4:3,6-dianhydrohexitol dihexanoate.

6. The composition as claimed in one of the preceding claims, in which the 1,4:3,6-dianhydrohexitol ester (A) is an isosorbide ester.

7. The composition as claimed in one of the preceding claims, in which the compound (B) is a 1,4:3,6-dianhydrohexitol diester or an ester of cyclohexanepolycarboxylic acid.

8. The composition as claimed in the preceding claim, in which the compound (B) is a 1,4:3,6-dianhydrohexitol diester, preferably an isosorbide diester.

9. The composition as claimed in one of claims 1 to 8, in which the amount of (A) ranges from 0.5% to 50% of the total weight of (A) and (B), advantageously from 1% to 25%, preferably from 2% to 20%, for example from 4% to 19%.

10. A polymer paste comprising a blend of a polymer powder and the composition as claimed in one of claims 1 to 9.

11. The use of a composition as claimed in one of claims 1 to 9 to plasticize a polymer.

12. The paste as claimed in claim 10, in which the polymer is chosen from vinyl polymers, such as polyvinyl chloride, polyurethanes, polyesters, cellulose polymers, starches, acrylic polymers, polyacetates and polyamides or a blend of these polymers, preferably polyvinyl chloride.

13. The use as claimed in claim 11, in which the polymer is chosen from vinyl polymers, such as polyvinyl chloride, polyurethanes, polyesters, cellulose polymers, starches, acrylic polymers, polyacetates and polyamides or a blend of these polymers, preferably polyvinyl chloride.

14. A process for the manufacture of an object based on a plasticized polymer composition, the latter comprising a polymer (C) and the constituents (A) and (B) as defined in one of claims 1 to 9, said process comprising:
• a stage of selecting the ester (A), the compound (B) and the polymer (C);
• a stage of introducing the constituents (A), (B) and (C) into a mixer system, the amounts of (A) and (B) being introduced in the proportions as defined in one of claims 1 to 9;
• a stage of mixing the constituents (A), (B) and (C) ;
• a stage of heating this blend;
• a stage of shaping the blend into the form of an object;
• finally, a stage of recovering said object comprising a plasticized polymer composition;
it being possible for the stage of introducing the constituents (A), (B) and (C) into the mixer to be carried out separately or via a blend of constituents, and simultaneously or sequentially;
it being possible for the mixing and heating stages to be carried out simultaneously or sequentially.

15. The process as claimed in claim 14, in which the blending stage is a stage of thermomechanical blending and the mixer system of which is a mixer for thermoplastics, preferably chosen from kneaders, Buss mixers, open mills and extruders.

16. The process as claimed in either of claims 14 and 15, in which the shaping stage is a calendering stage.

17. The process for the manufacture of the object as claimed in claim 14, **characterized in that**:
• the stage of blending the constituents (A), (B) and (C) is carried out in order to form a polymer paste;
• the shaping stage is a stage of coating, dipping, slushing or rotationally molding this polymer paste, in order to form a preformed object;
• the heating stage being a stage of curing said preformed object, which can take place during or after the shaping stage, in order to form the object.

18. The process as claimed in the preceding claim, **characterized in that** the shaping stage is a stage of coating on a support and that the heating stage is a curing stage which takes place after the coating stage.

19. The manufacturing process as claimed in one of claims 14 to 18, **characterized in that** the polymer is chosen from vinyl polymers, such as polyvinyl chloride, polyurethanes, polyesters, cellulose polymers, starches, acrylic polymers, polyacetates and polyamides and the blends of these polymers, preferably polyvinyl chloride.

20. The process as claimed in one of claims 14 to 19, **characterized in that** the constituents (A), (B) and (C) are introduced into the mixer system in proportions by weight such that the sum of (A) and (B) ranges from 1 to 900 parts per 100 parts of polymer (C), preferably from 10 to 120 parts of (A) and (B).

21. The use as polymer gelling accelerator of a 1,4:3,6-dianhydrohexitol ester (A), the molar mass of which ranges from 255 to 345 g.mol⁻¹, chosen from isosorbide, isomannide and isoidide monoesters and diesters.
